## Europäisches Patentamt

## European Patent Office

## Office européen des brevets

(11) Publication number: **0 097 517**
**B1**

## EUROPEAN PATENT SPECIFICATION

(45) Date of publication of patent specification: **24.06.87**

(21) Application number: **83303543.9**

(22) Date of filing: **20.06.83**

(51) Int. Cl.⁴: **A 61 F 13/00,** A 61 L 15/01, A 61 L 15/03

(54) Wound dressing, manufacture and use.

(30) Priority: **22.06.82 GB 8218088**

(43) Date of publication of application:
**04.01.84 Bulletin 84/01**

(45) Publication of the grant of the patent:
**24.06.87 Bulletin 87/26**

(84) Designated Contracting States:
**BE CH DE FR GB IT LI NL SE**

(56) References cited:
**EP-A-0 050 514
GB-A-1 310 460
GB-A-1 440 191
GB-A-2 093 702
GB-A-2 093 703
US-A-3 434 472
US-A-3 888 248**

(73) Proprietor: **Smith and Nephew Associated Companies p.l.c.
2, Temple Place Victoria Embankment
London WC2R 3BP (GB)**

(72) Inventor: **Lang, Stephen Michael
4 New Cottages Wicken Bonhunt
Nr. Saffron Walden Essex (GB)**
Inventor: **Webster, David Fitzgerald
10 Portland Road
Bishops Stortford Hertfordshire (GB)**

(74) Representative: **Cole, William Gwyn
Corporate Patents Department
Smith and Nephew Research Limited
Gilston Park
Harlow Essex CM20 2RQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an absorptive wound dressing suitable for use on burns or other wounds which dressing has a reduced tendency to adhere to the wound. The present invention also relates to the manufacture and use of such dressings.

Burns and other related wounds such as donor sites and the like present a serious problem in that they tend to produce large amounts of exudate which can cause conventional dressings to become saturated or to stick to the wound or even to become infected. One method of covering such wounds has been to cover the wound with a material into which new epithelial or fibroblast growth can penetrate. Dressings of this kind are disclosed in U.S. Patents Nos. 3526224, 3648692 and 3949742.

However such dressing can be extremely painful to remove and often require surgical excision. A fundamentally different approach requiring a fundamentally different type of dressing is to employ materials that are designed to reduce the propensity to adhere to the wound. Dressings of this kind are disclosed in British Patent No. 439085, French Patent No. 947609, United States Patents Nos. 3543750, 2923298 and British Patent No. 778813 which later patents cover successfully used materials such as Melolin ("Melolin" is a registered Trade Mark of T. J. Smith and Nephew Limited, Welwyn Garden City, Herts., U.K.). One more recent attempt at non-adherent dressings is United States Patent No. 3709221 which discloses a dressing having an outer microporous liquid repellent fibrous layer, an inner macroporous fibrous layer and an absorbent intermediate layer which was also envisaged as normally being fibrous. In order to reduce the tendency of this material to adhere to the wound the inner layer had to be treated with an agent to render it non-wetted by body liquid. It is now realised that it would be desirable to provide a dressing in which the wound facing layer did not require special treatment. As it will become apparent hereinafter it has now been discovered that by avoiding fibrous materials it is possible to produce a dressing with reduced tendency to adhere to wounds without the need for special treatments. An attempt at producing an absorbent dressing is described in U.S. Patent No. 3888248 which describes a dressing fabricated from at least four sheet materials. The wound facing part of the dressing apparently consists of a grid or scrim coated with polyethylene in such a manner that the polyethylene surrounds the filaments of the grid and collects any loose thread or particle that may be present in the core material. It is now realised that it is desirable to avoid the use of wound facing layers that can allow such penetration of the central layer to the wound surface. It has also be realised that it would be desirable to provide a material that was highly conformable to the wound so that it is possible to minimise the quantity of exudate between the wound surface and the dressing. U.S. Patents Nos. 3709221 and 3888248 disclose materials which are bonded along their edges which may reflect a desire to improve conformability. The dressing of the present invention allows for bonding over the whole of the operative area while retaining flexibility. European Application No. 0050514 discloses two layer sterile wound dressings consisting of elastomeric polyurethane net bonded to a fibrous absorbent layer. U.S. Patent No. 3927669 discloses a bandage which comprises a small foam pad covered by a perforated film wound facing layer and an adhesive coated perforated film backing layer which adheres the bandage to the body.

It has now been discovered that a low adherency wound dressing can be made which consists essentially of three layers and in which both outer layers can act as a wound facing.

The present invention provides a low adherency wound dressing which consists essentially of a laminate of a conformable hydrophilic foam absorbent layer between two conformable apertured outer layers characterised in that the two outer layers are coextensive with the absorbent layer and each outer layer comprises a conformable net of elastomeric polymer in which net the strand and junctures are formed integrally and in which the void area is 25% to 75%.

Normally the two outer conformable net layers are bonded to opposed faces of the conformable hydrophilic foam absorbent layer.

Either conformable net layer of the dressing of this invention can act as a low adherency wound facing layer. This layer allows wound exudate to pass to the absorbent layer but prevents the absorbent layer making direct contact with the wound surface.

The wound dressing of the invention has the advantage that either face of the dressing when applied to a wound surface will act as a low adherency wound facing layer, thus eliminating the possibility of a non-wound facing layer being applied to the wound surface, which can occur with a wound dressing having only one low adherency wound facing layer.

The two conformable net layers of the wound dressing can have a different construction but it is preferred that the two net layers have the same construction. Such preferred wound dressings of the invention having two outer conformable net layers of the same construction will have less tendency to delaminate or curl, for example on swelling, than a wound dressing having two outer conformable net layers of different construction.

The net used in this invention is a net with strands and junctures which have been formed integrally during manufacture.

Preferably the net of both outer layers is sufficiently conformable to allow the wound dressing to conform to the body contours and thereby maintain overall contact with the wound surface to ensure that exudate from the wound is absorbed.

It is also desirable that the net of both outer layers should be sufficiently elastically extensible to adjust to any dimensional changes in the absorbent layer which may occur, for example, expansion on liquid uptake.

Normally the net of both layers is made of a pharmaceutically acceptable water insoluble polymer. Suitable elastomers include polyurethanes, polybutadiene, polyether ester polyamide block copolymers, polyether ester polyester copolymers and the like.

The preferred material for the nets are thermoplastic polyurethanes.

Preferred thermoplastic polyurethanes are linear polyurethanes containing polyether or polyester groups. Suitable linear polyester polyurethanes are disclosed in U.S. Patent Specification No. 2,871,218. Suitable linear polyether polyurethanes are disclosed in U.S. Patent Specification No. 2,899,411. Favoured thermoplastic polyurethanes include Estanes from B. F. Goodrich Chemical Company. Preferred solution casting grades are Estane (Trade Mark) 5714F1, 5702, 5703 and 5707F1. Preferred extrusion grades are Estane 58201 and 58309.

Suitable polybutadienes are 1,2 polybutadienes. Favoured 1,2 polybutadienes contain a major amount of syndiotactic 1,2 polybutadiene, have a crystallinity of 25% to 30% and an average molecular weight in excess of 100,000. Preferred 1,2 polybutadienes are known as RB 810, RB 820 and RB 830 made by Japan Synthetic Rubber Company.

Suitable polyether ester polyamide elastomer block copolymers are linear polymers containing polyoxyalkylene blocks and polyamide blocks interconnected by ester groups. These polymers normally contain polyoxyalkylene blocks of molecular weight 200 to 6000 and polyamide blocks of molecular weight 300 to 15000. The polyoxyalkylene blocks can be polyoxyethylene, polyoxypropylene or poly (tetramethylene glycol) blocks or mixtures thereof. The polyamide blocks can be products of the polymerisation of cycloalkyl lactams having 6 to 12 carbon atoms for example a polyamide derived from nylons 6, 6-6, 6-9, 6-10, 6-12, 9-6 11 or 12.

Polyether ester polyamide block copolymers of this type are disclosed in British Patent No. 2063279A. Preferred polyether ester polyamide block copolymers are known as Pebax available from ATO Chemie (UK), Limited. A favoured polyether ester polyamide block copolymer is Pebax 2533SN00.

Suitable polyether ester polyester elastomer copolymers contain long chain polyether ester units derived from a high molecular weight polyether glycol and an aromatic dicarboxylic acid linked to short chain ester units derived from an aliphatic glycol and an aromatic dicarboxylic acid. Such polymers can contain polyoxyalkylene blocks of molecular weight 350 to 6000. Preferred polyether ester polyester copolymers contain poly (oxytetramethylene) glycol terephthalate blocks linked to tetramethylene terephthalate blocks. Polyether ester polyester copolymers of this type are disclosed in the Encyclopedia of Polymer Science and Technology Supplement Volume 2 pages 485 to 510, and in British Patent No. 1,404,925. Suitable polyether ester polyester copolymers for use in this invention include Hytrel polyester elastomers available from Dupont. A favoured Hytrel polyester elastomer is Hytrel grade, 4056.

The desirable conformability of the wound dressing of the invention is consistent with the use of elastomeric materials such as nets of polyurethane or other elastomer.

Suitable nets of polyurethane or other elastomer will have an elongation at break of 100% to 800%, desirably of 200% to 750% and preferably of 300% to 700% when measured as a 2.5 cm wide strip at 30 cm/min strain rate at 20°C.

The net of either layer of the wound dressing of the invention can have any convenient form depending on the chosen arrangement of strand, juncture and aperture areas and also their shapes and relative size.

The number and size of the apertures in the net will be sufficient to allow the wound exudate to pass through the net to the absorbent layer. Most aptly the net is adapted so that the size of apertures in combination with the thickness of the film prevent the absorbent layer contacting the wound surface. Suitable net have apertures with a dimension of from 0.05 to 4 mm, more aptly from 0.05 to 2.5 mm and preferably from 0.1 to 2.5 mm. Suitable nets have 2 to 40 apertures and preferably 4 to 24 apertures per cm. Favoured nets of the invention have 4 to 24 apertures per cm with a dimension of 0.05 to 2.5 mm. The wound face of the net suitable will have 25 to 75% of its area void (the apertures) and most suitably will have 35 to 65% of its area void. Suitable nets have a thickness of 0.01 to 2.5 mm and preferably a thickness of 0.05 to 0.5 mm.

The net used in this invention aptly will have a weight per unit area of 10 $g/m^2$ to 80 $g/m^2$ and preferably will have a weight of 15 $g/m^2$ to 50 $g/m^2$.

In one preferred form the net consists essentially of longitudinal and transverse strands intersecting at right angles to give a square grid hole pattern.

Variations on the square grid pattern can give other desirable forms of the integral net. Unequal density of strands in either the longitudinal or transverse directions will give rectangular hole areas. Continuous parallel strands in one direction with a staggered arrangement of connecting strands in the other direction will give a "brickwork" pattern. Other apt forms of the integral polymer nets can have strands at an angle to the longitudinal or transverse direction (that is diagonal strands). Another preferred form of integral net has two sets of diagonal parallel strands intersecting at right angles to give a "diamond" pattern of square shaped apertures. Another apt form of the integral polymer net can have a staggered arrangement of circular or approximately circular (for example hexagonal) arrangements of strands and hole areas. The integral polymer net can be in the form of a mixed pattern of two or more of the arrangements if desired.

Apt nets for use in the wound dressing of the invention are nets of polyurethane for example Estane 5714F with 4 to 8 per cm square apertures in particular 4, 6 or 8 per cm square apertures with an aperture size of 0.7 mm to 1.5 mm.

A favoured net for use in the wound dressing of the invention is a net of polyurethane for example Estane 5714F with 4 per cm square apertures with an aperture size of approximately 1.4 mm arranged in diagonal rows (45°).

The conformable hydrophilic polymer foam absorbent layer used in the dressing of this invention is adapted to be capable of absorbing the wound exudate e.g. from a burn. It is desirable that the hydrophilic foam layer absorbs the wound exudate rapidly as this enhances the low adherency properties of the dressing. Such rapid absorption prevents pooling of exudate between the dressing and the wound and it has been found that this prevention of pooling is desirable.

Suitable conformable hydrophilic foams will normally be flexible open cell foams. The open cells of the foam should extend in to its surface to ensure that the foam is capable of absorbing fluids in contact therewith. The ability of open cell foam to absorb and retain fluids depends to some extent on the size of foam cells and the porosity of the foam. Suitable open cell hydrophilic foams of dressings of the invention have a cell size of 30 microns to 700 microns and preferably a cell size of 50 microns to 500 microns. Apt open cell hydrophilic foams of dressings of the invention have 20% to 70% and preferably 30% to 60% of the total membrane area of the cells as membrane openings. Such open cell foams permit transport of fluid and cellular debris into and within the foam.

Apt foams may be polyurethane, carboxylated butadiene styrene rubber, polyacrylate or the like foam. Such foams may be made of hydrophilic materials per se or may be treated to render them hydrophilic, for example with surfactants. It is much preferred to use foams which are made of polymer which is itself hydrophilic as it has been found that the exudate is less likely to coagulate rapidly. The use of such foams of hydrophilic polymer in dressings of the invention can allow the wound to be maintained in a moist condition even when the exudate produced has been absorbed and removed from the wound surface.

Favoured hydrophilic polymer foams are hydrophilic polyurethane and especially those which are made of crosslinked hydrophilic polyurethane.

Preferred foams can be made by reacting a hydrophilic isocyanate terminated polyether prepolymer with water. Favoured hydrophilic polyurethane foams of this type include those known as Hypol foams. Hypol foams can be made from Hypol hydrophilic prepolymers marketed by W. R. Grace and Co.

Suitable hydrophilic foam absorbent layers have a thickness of 0.5 mm to 20 mm, more suitable 0.8 mm to 15 mm and preferably 1 mm to 12 mm.

Apt wound dressings of the invention comprise a hydrophilic polyurethane foam absorbent layer between two layers of polyurethane net.

The wound dressing of this invention may be in any convenient form. A preferred form is a pad of rectangular shape. Another preferred form is an elongate strip which may be in the form of a roll. Such a strip may be used as a bandage or may be used to prepare smaller dressings.

It is desirable that the wound dressings of the invention are sterile. The wound dressing of the invention is advantageously provided in bacteria impervious pouches. Such packed forms can be prepared under aseptic conditions or alternatively sterilised after packing by a conventional procedure. A favoured sterilisation procedure is heat sterilisation, for example by steam. Other favoured procedures are ethylene oxide sterilisation and gamma irradiation.

The wound dressing of the invention can contain a topically effective medicament. Most suitably the medicament is an antibacterial agent. Preferably the antibacterial agent is a broad spectrum antibacterial agent such as a silver salt such as silver sulphadiazine, and acceptable iodine source such as povidone iodine (also called polyvinyl pyrrolidone iodine or PVP/I), chlorhexidine salts such as the gluconate, acetate, hydrochloride or the like salts or quaternary antibacterial agents such as benzalkonium chloride or the like.

A preferred medicament for inclusion in the dressings of this invention is silver sulphadiazine. A further preferred medicament for inclusion in the dressing of this invention is chlorhexidine which will normally be present as one of its aforementioned salts.

The medicament may be present by 0.2% to 20%, more usually from 0.3 to 10% and preferably 0.5 to 5% by weight of the dressing, for example 1%, 1.2% or 3% and the like. The medicament is present in the invention in the foam layer.

The low adherency wound dressing of the invention can be made by a process which comprises forming a laminate of a conformable hydrophilic foam absorbent layer between two outer layers of conformable net as hereinbefore described.

The laminate can be formed by bonding the two outer net layers to opposed faces of the foam layer in one or more laminating processes. Suitable bonding methods include heat sealing and adhesive bonding. The preferred bonding method is heat sealing.

The net and foam layers can be heat sealed by heat and pressure in a conventional manner in one and more laminating processes. An apt heat sealing process comprises passing a net layer in contact with a foam layer through the nip of a heated metal roller and a rubber roller under low pressure. To ensure that the net layer is in a heat softened state it is preferred that the net layer contacts the heated metal roller. Thus by this method the laminate of the invention can be formed by heat sealing the two outer layers of net to opposed faces of the foam in two separate operations. Alternatively the two outer layers can be laminated to the foam layer in one process by passing the layers through the nips of two sets of rubber and heated metal rollers.

It is prefered that the net is supported on its embossed casting sheet as described hereafter during the heat seal lamination process. It has been found with this arrangement that the supported net has less tendency to be compressed and 'flattened' into the surface of the foam by the heat and pressure of the laminating process thus ensuring that the laminated net is a discrete layer on the foam surface.

If an alternative process the foam layer can be formed in contact with a net layer. The second net layer can then be bonded to the opposite face of the foam layer by heat sealing as described hereinbefore.

In a continuous process the wound dressing can be made in the form of a continuous strip which is then cut up into suitable size dressings.

The conformable hydrophilic polyurethane foam can be made by mixing together an isocyanate terminated polyether having functionality of more than two with a surfactant and water and casting the mixture onto a surface. This surface advantageously may be the wound facing layer of the dressing.

Preferred isocyanate terminated polyethers include Hypols FHP 2000, 2001, 3000, 3001, 2002 and 2000HD marketed by W. R. Grace and Co. Hypols are described in a booklet published by W. R. Grace and Co. "Hypol: foamable hydrophilic polymers—laboratory procedures and foam formulation". Their preparation and use are disclosed in British Patent Specifications Nos. 1429711 and 1507232.

Suitable surfactants for forming comformable hydrophilic polymer foams include non-ionic surfactants. Favoured non-ionic surfactants are oxypropylene—oxyethylene block copolymers known as Pluronics (Trade Mark) marketed by BASF Wyandotte. Preferred Pluronics include L64, F87, P38, P75 and L62. Another favoured non-ionic surfactant is a polyoxyethylene stearyl ether known as Brij 72 (Trade Mark) marketed by Honeywell Atlas.

To prepare a suitable foam 100 parts by weight of Hypol FHP 2000, 2001, 3000, 3001, 2002 or 2000HD is mixed with 0.3 to 7 parts by weight of surfactant or mixtures of surfactants and 30 to 300 parts by weight of water and the foaming mixture cast onto a surface. Typical foaming mixtures have a cream time of about 20 secs., a rise time of about 250 secs. and a cure time of about 400 secs.

In a continuous process for forming the foam the ingredients are fed into a continuous mixing and dispensing machine. Suitable conformable hydrophilic polymer foam layers can be made by casting the foaming mixture before it sets onto a suitable surface by means of a casting head.

A suitable mixing and dispensing machine is known as Vari-o-Mix supplied by Prodef Engineering Limited. The foam mix can conveniently be deliverd to the casting head by means of a "fishtail" die.

It is preferred that the foam is cast onto a release surface for example a silicone coated release paper. A suitable silicone coated release paper is known as Stearalese No. 46 available from Sterling Coated Papers Limited.

The nets of elastomer can be made by casting the elastomer in a flowable state onto a surface having a pattern of discrete raised areas and interconnected recessed areas and treating the cast net to form a solid integral net. The flowable state of the polyurethane can include solutions, dispersions, hot melts and powders which can be dried, coated, fused or otherwise to form a solid net. The casting surface may be in the form of a roller, an endless flexible belt or a length of sheet material. It is preferred that the casting surface has release properties to enable the formed net to be removed from the casting surface. The pattern of the discrete raised areas and interconnected recessed areas on the casting surface selected dictates the structure of the resulting net.

Processes for making the conformable nets of the wound dressing of the invention are disclosed in European Patent Application No. 82300715.8 (Publication No. 0059048).

A preferred method of making the integral nets of polyurethane is by casting a solution of a thermoplastic polyurethane onto a melt embossed polyolefin sheet and drying the cast net in a hot oven.

Suitable casting solutions can contain 15% to 35% by weight of thermoplastic polyurethane, preferably 20% to 30% by weight. Favoured casting solutions contain 20 to 25% by weight of Estane 5702 or Estane 5703 in acetone. Another favoured solution contains 20 to 30% by weight of Estane 5714F in tetrahydrofuran or mixture of tetrahydrofuran and acetone.

Analogous procedures may be used to prepare nets from other elastomers.

The melt embossed polyolefin sheet can be made by the method given in British Patent Specification No. 1055963. A suitable embossed polyolefin sheet has a pattern of 8 per cm raised areas in the form of square truncated pyramids 1 mm wide and 0.5 mm high with sides sloping to a 60° conical angle and longitudinal and transverse square grid recesses 0.25 mm wide at the base and 0.75 mm at the top. A favoured embossed polyolefin sheet has a pattern of 6 per cm raised areas in diagonal rows (45°) of square truncated pyramids 1.35 mm wide at their base, 0.7 mm wide their top and 0.45 mm high with sides sloping to a 70° conical angle.

A preferred embossed polyolefin sheet has a pattern of 4 per cm raised areas in diagonal rows (45°) of square truncated pyramids 2 mm wide at their base, 1.425 mm wide at their top and 0.5 mm high with sides sloping to a 60° conical angle.

Suitable polyolefins for the melt embossed sheet include high density polyethylene, polypropylene and copolymers of propylene and ethylene.

It is one of the surprising features of this invention that antibacterical agent can be incorporated into a hydrophilic polyurethane foam and will thereafter be available to aid in maintaining the wound to which the dressing is applied free of infection.

It is particularly surprising that medicaments such as silver sulphadiazine and chlorhexidine hydrochloride and the like can be incorporated into the proto foam prior to polymerisation since the presence of compounds containing basic nitrogen atoms may well have been expected to radically change the nature of the foam which has now been found not to occur.

The medicament may be introduced into the foam either by incorporation prior to foaming or by incorporation into the intact foam which has previously been prepared.

If the medicament is to be introduced prior to foaming then the medicament must either be free of reactive moieties which would react with the components of the mixture to be foamed (for example it must not contain free amino groups which could react with the isocyanates present) or else the medicament must be of low solubility so that its potential reactivity is suppressed. Thus for example medicaments such as silver sulphadiazine and chlorhexidine hydrochloride are easily incorporated into the foam by dispersing the desired amount of the medicament into the prepolymer mixture, for example dispersing it within the aqueous solution of the surfactant before mixing with the isocyanate containing materials. Most suitably the insoluble medicaments are in finely divided form and are most preferably micronised.

It has been found that more soluble salts such as chlorhexidine gluconate cannot be incorporated in this fashion since reaction with prepolymer components can occur and a more rigid and antibacterically ineffective foam results. Fortunately it has now been discovered that soluble medicaments can be included into the foam after it has been prepared by soaking the foam in a solution of the medicament.

As previously indicated hereinbefore the dressings of this invention may be adapted to release antibacterically effective amount of antibacterial agent into the wound covered by the dressing. Thus in an alternative aspect this invention provides a method of treating a wound so as to aid in rendering or maintaining it free of infection which comprises contacting the wound with a dressing of this invention adapted to release an antibacterical agent. Most aptly this aspect of the invention is employed in rendering or maintaining burns free of infection. The antibacterial agent present is favourably a silver salt such as silver sulphadiazine or a chlorhexidine salt such as chlorhexidine hydrochloride or a mixture thereof. Preferably the antibacterial agent present is silver sulphadiazine.

The absence of fibres in the dressing enhances the non-adherent properties of the wound dressings of the invention.

Example.
Preparation of polyurethane net (4 apertures/cm)

A 20% solution of a polyurethane (Estane 5714F) in a 60/40 (weight by weight) mixture of tetrahydrofuran/acetone was cast into the recesses of a 15 cm wide melt embossed polypropylene sheet (polypropylene containing 40% by weight chalk filler ref PXC 4999 available from ICI Plastics Limited) by means of a blade of over soft bed coating unit to form a cast integral net. The polypropylene sheet had a melt embossed pattern of 4 per cm raised areas in diagonal rows (45°) of square truncated pyramids 2 mm wide at their base, 1.4 mm wide at their top and 0.5 mm high with sides sloping to a conical angle of 60°. The cast polyurethane net on the embossed film was dried by passage through a hot air oven at a temperature of 80°C for two minutes. The net had a weight per unit area of 33 $g/m^2$ and 4 per cm square apertures of approximately 1.4 mm in size arranged in diagonal rows (45°).

Preparation of the conformable hydrophilic polyurethane foam absorbent layer

Using a two component dispensing unit (Vari-o-Mix supplied by Prodef Engineering Limited) a foaming mixture was formed by mixing Hypol FHP 2002 and Brij 72 (1% aqueous solution) in the ratio of 1:2. The foaming mixture was fed into the coating head by means of an output nozzle in the form of a 15 cm 'fishtail die' and coated onto silicone coated release paper (Stearalese No. 46 available from Sterling Coated Papers Limited) by means of a knife over roller coating head set at a gap of 1 mm. The cast foam was dried by passage through an air circulating oven at a temperature of 50°C for 5 minutes. The cast foam had a thickness of 2 mm.

Preparation of the low adherency wound dressing

The conformable hydrophilic polyurethane foam on its silicone coated release casting paper was heat laminated to the conformable polyurethane net (4 apertures/cm) on its embossed coasting sheet by passing the layers between the nip of a silicone rubber roller and a steel roller heated by circulating oil to a temperature of approximately 135°C. The embossed sheet carrying the polyurethane net was fed in against the heated steel roller to ensure that the net was heated to its softening temperature prior to its lamination to the foam.

The silicone coated release paper was then removed from the foam and a second layer of polyurethane net (4 apertures/cm) on its embossed casting sheet was heat laminated to the foam surface by a similar heat laminating process.

The embossed carrier sheets were then removed from the net surfaces to give a 15 cm wide laminate strip suitable for making the low adherent wound dressings of the invention.

The laminate consisted of a conformable hydrophilic polyurethane foam absorbent layer between and bonded to two outer layers of polyurethane net.

The laminate had a thickness of approximately 2 mm and a weight per unit area of approximately 500 g/m$^2$.

The laminate strip was cut into 10 cm×15 cm sheets to give low adherency wound dressings of the invention.

Demonstration of effectiveness

The suitability of using a three layer net/foam/net laminate to form the low adherency wound dressing of the present invention was demonstrated as follows, using as a comparison a two layer net/foam laminate. A net/foam laminate comprising an elastomeric net bonded to a hydrophilic foam was prepared by casting a polyurethane net and a polyurethane foam as described above and then bonding the net to the foam by passing the two layers between the nip of a silicone rubber roller and a steel roller as described above. A circular piece of the laminate which was 4.5 cm in diameter and 2.1 mm in thickness was cut from the strip of laminate formed. A circular piece of laminate 4.5 cm in diameter and 2.8 mm in thickness was cut from a net/foam/net laminate formed in the manner described above. The net was a polyurethane net and the foam a polyurethane foam of the same type as used in the two layer laminate. The net face of each circular piece of laminate was allowed to stand in contact with 10 ml of horse serum. After reaching equilibrium in regard to absorption of serum, the effect on each circular piece of laminate was determined. It was observed that the net/foam laminate had assumed a dome shape in which the centre of the laminate had risen 8 mm from its flat position. The surface of the foam which was not bonded to net had expanded whilst the other surface, although bonded to an elastic net had not been allowed to expand equally. The resulting tension caused the laminate to change shape. In practice this would have removed the net surface and thereby the absorbent from the exuding wound and so impaired further absorption. By contrast it was observed that the net/foam/net laminate had remained substantially flat, although absorbing approximately the same amount of serum, 6.2 g of serum per g. of laminate. The only observed effect was the raising of the periphery of the dressing by approximately 1 mm. However it was clear that a dressing formed from the net/foam/net laminate would remain in contact with the surface to which it was applied after absorbing for example wound exudate. The presence of elastic net on both sides of the foam allowed the foam to expand evenly and did not impair its absorbing capacity.

## Claims

1. A low adherency wound dressing which consists essentially of a laminate of an absorbent layer of conformable hydrophilic foam between two conformable apertured outer layers characterised in that the two outer layers are coextensive with the absorbent layer and each outer layer comprises a conformable net of elastomeric polymer in which net the strands and junctures are formed integrally and in which the void area is 25% to 75%.

2. A low adherency wound dressing as claimed in claim 1 in which the two outer layers of conformable net have the same construction.

3. A low adherency wound dressing as claimed in claim 2 in which the net has intersecting diagonal strands which form a diamond pattern.

4. A low adherency wound dressing as claimed in any of claims 1 to 3 in which the elastomeric polymer comprises polyurethane.

5. A low adherency wound dressing as claimed in any of claims 1 to 4 in which the two outer layers of conformable net have 2 to 40 apertures per cm with a dimension of from 0.05 to 4 mm.

6. A low adherency wound dressing as claimed in any of claims 1 to 5 in which the conformable hydrophilic foam is a foam of a hydrophilic polymer.

7. A low adherency wound dressing as claimed in claim 6 in which the hydrophilic polymer is a hydrophilic polyurethane.

8. A low adherency wound dressing as claimed in any of claims 1 to 7 in which the hydrophilic foam comprises an open cell foam with a cell size of 50 microns to 500 microns.

9. A low adherency wound dressing as claimed in any of claims 1 to 8 in which the hydrophilic foam comprises an open cell foam in which 30% to 60% of the total membrane area of the cells are membrane openings.

10. A low adherency wound dressing as claimed in any of claims 1 to 9 in which the dressing contains a topically effective medicament.

11. A sterile low adherency wound dressing as claimed in any of claims 1 to 10 within a bacteria impervious pack.

## Patentansprüche

1. Wundverband geringer Haftung, der im wesentlichen aus einem Schichtstoff aus einer absorbierenden Schicht aus einem schmiegsamen hydrophilen Schaum zwischen zwei schmiegsamen mit Öffnungen versehenen äußeren Schichten besteht, dadurch gekennzeichnet, daß die beiden äußeren Schichten von gleichen Ausdehnung sind wie die absorbierende Schicht und jede äußere Schicht ein schmiegsames Netz aus elastomerem Polymer umfaßt, wobei die Fäden und Verbindungsstellen des Netzes einstückig ausgebildet sind und der offene Bereich 25—75% beträgt.

2. Wundverband geringer Haftung nach Anspruch 1, bei dem die beiden äußeren Schichten aus schmiegsamen Netz die gleiche Konstruktion aufweisen.

3. Wundverband geringer Haftung nach Anspruch 2, bei dem das Netz kreuzende diagonale Fäden aufweist, die ein Diamantmuster bilden.

4. Wundverband geringer Haftung nach irgendeinem der Ansprüche 1—3, bei dem das elastomere Polymer Polyurethan umfaßt.

5. Wundverband geringer Haftung nach irgendeinem der Ansprüche 1—4, bei dem die beiden äußeren Schichten aus schmiegsamem Netz 2—40 Öffnungen/cm mit einer Abmessung von 0,05—4 mm aufweisen.

6. Wundverband geringer Haftung nach irgendeinem der Ansprüche 1—5, bei dem der schmiegsame hydrophile Schaum ein Schaum aus einem hydrophilen Polymer ist.

7. Wundverband geringer Haftung nach Anspruch 6, bei dem das hydrophile Polymer ein hydrophiles Polyurethan ist.

8. Wundverband geringer Haftung nach irgend einem der Ansprüche 1—7, bei dem der hydrophile Schaum einen offenzelligen Schaum mit einer Zellgröße von 50—500 µm umfaßt.

9. Wundverband geringer Haftung nach irgendeinem der Ansprüche 1—8, bei dem der hydrophile Schaum einen offenzelligen Schaum umfaßt, bei dem 30—60% des gesamten Membranbereiches der Zellen Membranöffnungen sind.

10. Wundverband geringer Haftung nach irgendeinem der Ansprüche 1—9, der ein örtlich wirksames Medikament enthält.

11. Steriler Wundverband geringer Haftung nach irgendeinem der Ansprüche 1—10 in einer für Bakterien undurchringlichen Verpackung.

**Revendications**

1. Pansement pour plaie d'adhérence faible qui consiste essentiellement en une stratification comprenant une couche absorbante de mousse hydrophile entre deux couches extérieures ouvertes adaptables caractérisé en ce que les deux couches extérieures sont coextensives avec la couche absorbante et chaque couche extérieure comprend un filet adaptable de polymèr élastomère, filet dans lequel les fils et les intersections sont formés intégralement et dans lequel la surface vide est de 25% à 75%.

2. Pansement pour plaie d'adhérence faible selon la revendication 1 caractérisé en ce que les deux couches extérieures du filet adaptable ont la même construction.

3. Pansement pour plaie d'adhérence faible selon la revendication 2 caractérisé en ce que le filet a des fils intersectant diagonalement, ceux-ci formant un motif en diamant.

4. Pansement pour plaie d'adhérence faible selon l'une quelconque des revendications 1 à 3 caractérisé en ce que le polymère élastomère comprend du polyuréthane.

5. Pansement pour plaie d'adhérence faible selon l'une quelconque des revendications 1 à 4 ccaractérisé en ce que les deux couches extérieures de filet adaptable ont de 2 à 40 ouvertures par centimètre ayant chacune une dimension de 0,05 à 4 mm.

6. Pansement pour plaie d'adhérence faible selon l'une quelconque des revendications 1 à 5 caractérisé en ce que la mousse hydrophile adaptable est une mousse de polymère hydrophile.

7. Pansement pour plaie d'adhérence faible selon la revendication 6 caractérisé en ce que le polymère hydrophile est un polyuréthane hydrophile.

8. Pansement pour plaie d'adhérence faible selon l'une quelconque des revendications 1 à 7 caractérisé en ce que la mousse hydrophile comprend une mousse à cellules ouvertes dont la dimension des cellules est de 50 à 500 microns.

9. Pansement pour plaie d'adhérence faible selon l'une quelconque des revendications 1 à 8 caractérisé en ce que la mousse hydrophile est formée d'une mousse à cellules ouvertes dans laquelle 30 à 60% de la zone membranaire totale sont des ouvertures de membrane.

10. Pansement pour plaie d'adhérence faible selon l'une quelconque des revendications 1 à 9 caractérisé en ce que le pansement contient un médicament actif localement.

11. Pansement stérile pour blessures d'adhérence faible selon l'une quelconque des revendications 1 à 10 caractérisé en ce qu'il est dans un emballage étanche aux bactéries.